# EUROPEAN PATENT SPECIFICATION

(11) **EP 4 075 440 B1**
(45) Date of publication and mention of the grant of the patent: **16.10.2024**
(21) Application number: 21168591.2
(22) Date of filing: 15.04.2021
(51) Int. Cl.: G16H 20/17, G16H 20/60, G16H 40/63

(54) **MEAL BOLUS SUBCATEGORIES IN MODEL BASED INSULIN THERAPY**
MAHLZEITBOLUSUNTERKATEGORIEN IN DER MODELLBASIERTEN INSULINTHERAPIE
SOUS-CATÉGORIES DE BOLUS DE REPAS DANS L'INSULINOTHÉRAPIE SE BASANT SUR UN MODÈLE

(43) Date of publication of application: 19.10.2022
(73) Proprietor: Insulet Corporation, Acton MA 01720 (US)
(72) Inventor: METZMAKER, Thomas, Harvard, MA 01451 (US); CONTE, Nicholas, Harvard, MA 01451 (US); MCLAUGHLIN, Ian, Groton, MA 01450 (US); LEE, Joon Bok, Acton, MA 01720 (US); BENTE, Paul Frederick, Acton, MA 01720 (US); ZADE, Ashutosh, San Diego, CA 92128 (US); CARDINALI, Steven, Tewksbury, MA 01876 (US)
(74) Representative: Peterreins Schley

(56) References cited:
- US-A1- 2020 219 625
- US-A1- 2021 050 085

## Description

### BACKGROUND

Due to the complicated and dynamic nature of the human body's response to insulin, patients may end up in a hypoglycemic or hyperglycemic state after being treated with insulin therapy. This outcome is undesirable for many reasons: hypoglycemia creates an immediate risk of a severe medical event (seizure, coma, death) while hyperglycemia creates long term negative health effects as well as the risk of ketoacidosis. Whether a person ends up in one of these states depends on a combination of data inputs and responses.

Mealtime is particularly difficult for diabetic users to manage because users have to estimate an amount of carbohydrates a meal contains and estimate an amount of insulin to deliver to mitigate the effects of the meal. While a closed loop system reduces the amount of engagement required from users, users typically still predict an amount of insulin to be delivered as a bolus to compensate for the meal and preemptively deliver insulin before the carbohydrates in the food making up the meal are absorbed.

It would be beneficial to mitigate this need for prediction and minimizing user involvement.

US 2020/219625 A1 discloses systems and methods for extracting blood glucose patterns and suggesting a behavior which may include receiving, at a computing device comprising a processor, temporal data including information regarding glucose readings; identifying, by the computing device, at least one pattern based on metabolite levels extracted from the temporal data the model including variables corresponding to each of the patterns; formulating, by the computing device, a model for predicting a metabolic response; and storing the model on a data storage device. Based on the model, the behavior may be suggested to maintain a blood glucose level within a desired range.

US 2021/050085 A1 discloses systems, devices and methods which are provided for determining a medication dose for a patient or user. The dose determination can account for recent and/or historical analyte levels of the patient or user. The dose determination can also take into account other information about the patient or user, such as physiological information, dietary information, activity, and/or behavior. Many different dose determination embodiments are set forth, pertaining to a wide array of different aspects of the system or environment in which the embodiments can be implemented.

### SUMMARY

Disclosed is an example of a non-transitory computer readable medium embodied with programming code executable by a processor. The processor when executing the programming code is operable to perform functions. The processor may receive information related to ingestion of a meal. The information may include a coarse indication of a size of the meal, a relative time of ingestion of the meal, and a general composition indication of the meal. A meal model may be selected from a number of meal models. A setting for a delay parameter may be determined based on the received relative time of ingestion of the meal, a setting for an extend parameter may be determined based on the received general composition indication of the meal, and a setting for a delivery constraint may be determined based on the received coarse indication of the size of the meal. The selected meal model may be modified using the determined setting for the delay parameter, the determined setting for the extend parameter, and the determined setting for the delivery constraint. A dose of insulin to be delivered in response to the received information may be determined using the modified, selected meal model. An instruction may be output for delivery to a drug delivery device. The outputted instruction may indicate a determined dose of insulin to be delivered.

An example of a device is disclosed that includes a processor and a memory. The memory may be operable to store programming code, an artificial pancreas application, and data related to the artificial pancreas application. The programming code and the artificial pancreas application are executable by the processor. The processor when executing the artificial pancreas application may be operable to control delivery of insulin, and to perform functions. The processor may receive information related to ingestion of a meals. The information may include a coarse indication of a size of the meal, a relative time of ingestion of the meal, and a general composition indication of the meal. A blood glucose measurement value that was received within a predetermined time range of the relative time of ingestion of the meal may be identified. A meal model may be selected from a number of meal models based on the identified blood glucose measurement value. A setting for a delay parameter may be determined based on the received relative time of ingestion of the meal. A setting for an extend parameter may be determined based on the received general composition indication of the meal. A setting for a delivery constraint may be determined based on the received coarse indication of the size of the meal. The selected meal model may be modified using the determined setting for the delay parameter, the determined setting for the extend parameter, and the determined setting for the delivery constraint. a dose of insulin to be delivered in response to the received information may be determined using the modified, selected meal model. An instruction that indicates a determined dose of insulin to be delivered may be output for delivery to a drug delivery device.

### BRIEF DESCRIPTION OF THE DRAWINGS

FIG. 1 illustrates an example of a process for updating model parameters related to a meal and implementing actions based a model using the updated model parameters.
FIG. 2 illustrates another flowchart of an example process implemented by an artificial pancreas application using the techniques described in the example of FIG. 1.
FIG. 3 is graph illustrating blood glucose profiles with respect to high absorbing food and slow absorbing food.
FIG. 4 illustrates a functional block diagram of a drug delivery system suitable for implementing the example processes and techniques described herein.
FIG. 5 illustrates an example of a graphical user interface presented on a user interface as described with reference to the examples of FIGs. 1-4.

### DETAILED DESCRIPTION

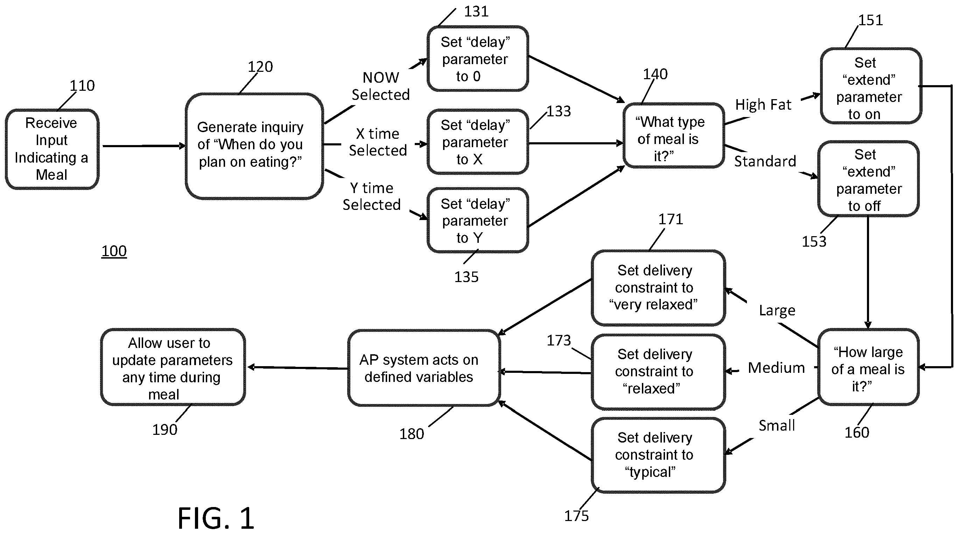

One or more examples provide a process that may be used with or by any additional algorithms or computer applications. The additional algorithms or computer applications may be referred to as an "artificial pancreas" algorithm-based system, or more generally, an artificial pancreas (AP) application, that provides automatic delivery of an insulin based on a blood glucose sensor input, such as that received from a CGM or the like. Examples of an artificial pancreas (AP) application as described herein may manage blood glucose levels and provide insulin therapy. The AP application may be a third-party artificial pancreas application that allows data provided by algorithms executing the process examples described herein In an example, the artificial pancreas (AP) application when executed by a processor may enable a system to monitor a user's glucose values, determine an appropriate level of insulin for the user based on the monitored glucose values (e.g., blood glucose concentrations or blood glucose measurement values) and other information, such as user-provided information, such as carbohydrate intake, exercise times, meal times or the like, and take actions to maintain a user's blood glucose value within an appropriate range. The appropriate blood glucose value range may be considered a target blood glucose value of the particular user. For example, a target blood glucose value may be acceptable if it falls within the range of approximately 80 mg/dL to approximately 120 mg/dL, which is a range satisfying the clinical standard of care for treatment of diabetes. However, an AP application as described herein may be able to establish a target blood glucose value more precisely and may set the target blood glucose value at, for example, 110 mg/dL, or the like. As described in more detail with reference to the examples of FIGs. 1-5, the AP application may utilize the monitored blood glucose values and other information to generate and send a command to a medical device including, for example, a pump, to control delivery of insulin to the user, change the amount or timing of future doses, as well as to control other functions based on user history, such as, for example, the modification of constraints on the amount or timing of doses of insulin, generation of prompts, receipt of inputs from a user, or the like.

To mitigate the need for user prediction and enable minimal user involvement, the disclosed examples provide a device, a system, a computer application or algorithm, computer-readable medium product and techniques that utilize meal models to address eating carbohydrates at mealtime. The use of a meal model is advantageous because it reduces user involvement as well as the burden on the user of estimating carbohydrates in a meal and calculating a resulting meal bolus dosage. In addition, the described examples also may enable automates processes to treat increasing blood glucose measurement values more aggressively without the user having to fear a hypoglycemic episode, which may happen with a mistaken estimate of an amount of carbohydrates in a meal or an amount of insulin in a meal bolus.

The meal models described in the following examples may be broken down into several categories based on a type of meal, timing of the meal, and a size of the meal. For example, the type of meal may be the composition of the meal and may be broken down as high fat, low fat or standard. Timing of the meal may be a meal that is in progress, in 15 minutes, in 30 minutes, or the like. In the examples, the size of meal may be broken up into coarse descriptions (e.g., small, medium, or large) rather than a specific estimate of an amount of carbohydrates. The example information allows the algorithm that controls insulin delivery to adjust a meal model to be more aggressive with insulin delivery by, for example, knowing what portion of the mealtime should be extended, when to begin delivery, and when to return to the standard basal model for insulin delivery.

In the examples, the information provided by a user may include the timing of the meal with reference to an input to the AP application. For example, a graphical user interface presented on a user interface of a PDM or other device may enable inputs related to the timing of the meal, such as "eating now," "started eating X minutes ago," "expect to eat in Y minutes," "finished eating U minutes ago," or the like. The AP application may, for example, in response to receiving, the input from the graphical user interface and estimate a blood glucose measurement value that a continuous blood glucose device is expected to provide after ingestion of the meal based on information related to the meal. In an example, the continuous blood glucose device may provide a blood glucose measurement value every 5 minutes to the AP application. The effects of the meal on the blood glucose measurement values may not be noticeable until after several blood glucose measurements are received. For example, the time between ingesting a meal and a change in blood glucose measurement values may be several minutes as the user's blood glucose must react to the ingestion of carbohydrates, and that reaction may take time to manifest itself in the change in blood glucose measurement values. The AP application may determine an amount of time needed to identify a change in blood glucose measurement values. After the determined amount of time, the AP application may compare the estimated blood glucose measurement value to a blood glucose measurement value received at the expected time. Based on the result of the comparison, the AP application may confirm whether the meal was ingested according to the input received from the graphical user interface or whether the input received from the graphical user interface was in error.

The AP application may conclude with providing increased insulin delivery as a reaction to increasing blood glucose measurement values that are greater than normal with the knowledge the user had recently eaten. The magnitude of the reaction allowable would be determined by the selected meal model and known metrics related to the user, such as Total Daily Insulin (TDI), Correction Factor, and Carbohydrate Ratio. Based on results of the described processes, the initial treatment target (e.g., amount of insulin to be delivered as a meal bolus in response to a meal as well as subsequent basal insulin deliveries) may also be adjusted to compensate for the greater insulin onboard (IOB) during this period. For example, the immediate bolus size versus extended bolus size and length may also be predicted using the selected meal model.

In addition, feedback in the form of blood glucose measurement values provided by a continuous blood glucose monitor (CGM), for example, may be tracked and the meal models may be further tailored to the individual based on the feedback results to become more effective over time.

This approach may make meal bolusing a less stressful experience for the user and allow the closed loop system to do more of the therapy adjustment rather than depending on the user for accurate meal estimation. An advantage of the described examples is the elimination of a user having to estimate a bolus amount for meals thereby enhancing the user's experience with the described AP application or a closed-loop insulin treatment system.

FIG. 1 illustrates an example of a process for updating model parameters related to a meal and implementing actions based on a model using the updated model parameters. For example, the AP application may implement a model when calculating bolus insulin doses. The model may have different parameters that may be set based on one or more of: a user's activity, user insulin delivery history, a user's blood glucose measurement value history, a user's insulin sensitivity, a user's insulin-to-carbohydrate ratio, or the like.

In the process 100, a user may be presented with a graphical user interface operable to receive inputs related to a user's mealtime experience. For example, the graphical user interface may have a "meal" button or some other input device, such as a microphone, that when actuated indicates to the AP application that the user is considering a meal. As shown at 110, a meal indication input may be received via the graphical user interface by the AP application. At 120, in response to receiving the input indicating a meal is imminent, the AP application may be operable to generate an inquiry that is output via the graphical user interface, requesting an indication of when the user plans to eat. For example, the prompt may be "When do you plan on eating?" or something to that effect. For the convenience of the user, the AP application may output via the graphical user interface several choices for selection by the user, such as "Now," "In X time," or "In Y time," or the like. The "X time" and "Y time" may be predetermined times, such as 15, 30, 45 or the like, that are in units of minutes or hours. Depending upon which prompt is selected, the AP application may take, for example, one of three actions at 131-135 with respect to delaying delivery of a bolus dosage related to the meal. In the example of FIG. 1, the AP application may in response to receipt of a relative time of ingestion of the meal set a delay parameter. For example, the AP application may receive a relative time indication of "Now," via an input to graphical user interface, and in response the delay parameter may be set by the AP application to a zero time delay (131). Alternatively, in response to a "X time" being selected, the AP application set a delay parameter equal to the time represented by "X time" (133). As a further alternative, the AP application set a delay parameter equal to the time represented by "Y time" in response to the selection of the "Y time" prompt (135). The delay parameter setting may be an amount of time that the AP application may either generate instructions for or that is included in instructions to an automated medical device or drug delivery device to deliver a meal bolus.

The broadest category in which the user may report meal size while still maintaining granularity is by announcing whether the meal is "fast absorbing" or "slow absorbing" through a simplified meal button or "small," "medium," or "large" on a sliding scale. In an example, a slider or any number of indicators can be used instead of a meal button. The meal size and composition can either be detected, or announced by user via an input device, in a coarse form. For example, after the AP application sets a delay parameter at one of 131, 133 or 135, the AP application may at 140 generate a prompt inquiring as to "what type of meal" the user is eating. The inquiry at 140 enables the AP application to determine whether the delay parameter needs to be extended for additional time depending on the type of meal. The type of meal may refer to the general composition of the meal, where composition may be broadly described as "low fat," "high fat," "standard," or the like. For example, the AP application may, in response to receipt of a general composition indication of the meal, determine a setting for an extend parameter. For example, if the meal is a "high fat" meal (e.g., a meal with a large amount of processed ingredients, a fast food hamburger or the like), the graphical user interface may enable an input indicating the meal is a "high fat" meal. The AP application in response to receiving a "high fat" meal indication, may set the extend parameter to ON (151). In addition to the "high fat" meal prompt, the AP application may be operable to generate a "standard" meal prompt on the graphical user interface. In response to an input indicating the "standard" meal prompt was selected instead of the "high fat" meal prompt, the AP application may set the extend parameter to OFF (153).

The extend parameter may be an additional model parameter that may be set to a numerical value to indicate when the user's blood glucose measurement values are expected to begin showing the user's reaction to ingesting the carbohydrates associated with the indicated meal. The extend parameter may be a further time delay in addition to delay parameter set at one of steps 131, 133 or 135. In an example, the extend parameter may be varied in cases of meals with similar carbohydrate but different fat or protein contents, which may impact the absorption rate of the meals and thus the rate of appearance of the impact of the carbohydrates.

After setting the extend parameter at either 151 or 153, the process 100 proceeds to 160. At 160, the AP application may be operable to generate a prompt via the graphical user interface that inquires as the size of the meal. For example, after a response to the prompt requesting an indication of a type of meal at 140, the graphical user interface may present another prompt inquiring as to "how large of a meal is it?" at 160. Of course, how "large" a meal is relative to each user so the AP application may use historical settings customized to the respective user when setting model parameters. The graphical user interface may present optional user interactive devices, such as buttons or a slider, that enable a user to input a size of the meal as small, medium or large.

In the example, the AP application may determine a setting for a delivery constraint based on a coarse indication of the size of the meal received in response to the query at 160. For example, the input at 171 may be an indication that the meal was "large," the AP application may be operable to set the delivery constraint to "very relaxed." While, in response to the AP application receiving an indication that the meal was "medium," the AP application may be operable to set the delivery constraint to "relaxed" (173). The AP application may be operable to set the delivery constraint to "typical" (175). The delivery constraint may, for example, be a maximum level of insulin that may be delivered in a given time period, such as a mealtime bolus, an amount of insulin equal to total daily insulin quantity, or the like. The delivery constraint may be implemented for the safety of the user to prevent an overdose of insulin, which may result in hypoglycemia or hyperglycemia. In the example, the "relaxation" of the delivery constraint may be an increase in the amount of insulin that may be delivered to compensate for the ingestion of the size of the meal indicated by either the large, medium or small meal identification. In an example, in response to an indication at 160 of a large meal being eaten, the model implemented by the AP application may calculate a bolus insulin dosage that may be close to, or may exceed, a delivery constraint for a maximum daily dosage of insulin to be delivered. As a result of the calculated bolus insulin dosage being close to the delivery constraint, the AP application may increase the maximum daily dosage a fixed percentage (i.e., relax the safety constraint) that is commensurate with the ingestion of a large meal.

The AP application at 180 may be operable to modify selected meal model using the determined setting for the delivery parameter, the determined setting for the delay parameter, and the determined setting for the delivery constraint, and utilize the modified, selected meal model to determine a dose of insulin to be delivered in response to the received information. For example, the determined settings for the delay parameter, the extend parameter and the delivery constraint may be used by the AP application in a calculation of an amount (or dosage) of insulin to be delivered as a meal bolus. The AP application may be operable to generate instructions and output the generated instructions to an automated medical device, such as a pod or other drug delivery device instructing delivery of the meal bolus at a predetermined time. Alternatively, the AP application may be operable to generate a presentation of the calculated insulin dosage on a graphical user interface of a computing device. For example, the AP application may provide the presentation of the calculated insulin dosage to allow a user to self-deliver the meal bolus as in an open-loop system.

In addition, the AP application when cooperating with an automated medical device, may wait for a period of time based, for example, on the delay parameter setting (which was set at one of 131, 133 or 135). During the period of time (i.e., the delay) in delivering the meal bolus, the AP application may request confirmation of previously selected inputs, such as those provided at 131, 133, 135, 151, 153, 171, 173 or 175. In addition, the AP application may allow a user to update parameters any time during a meal (190). For example, the user may change their mind during a meal and modify the chosen meal, decide not to eat as much, the like after inputting information at steps 131, 133, 135, 151, 153, 171, 173 or 175.

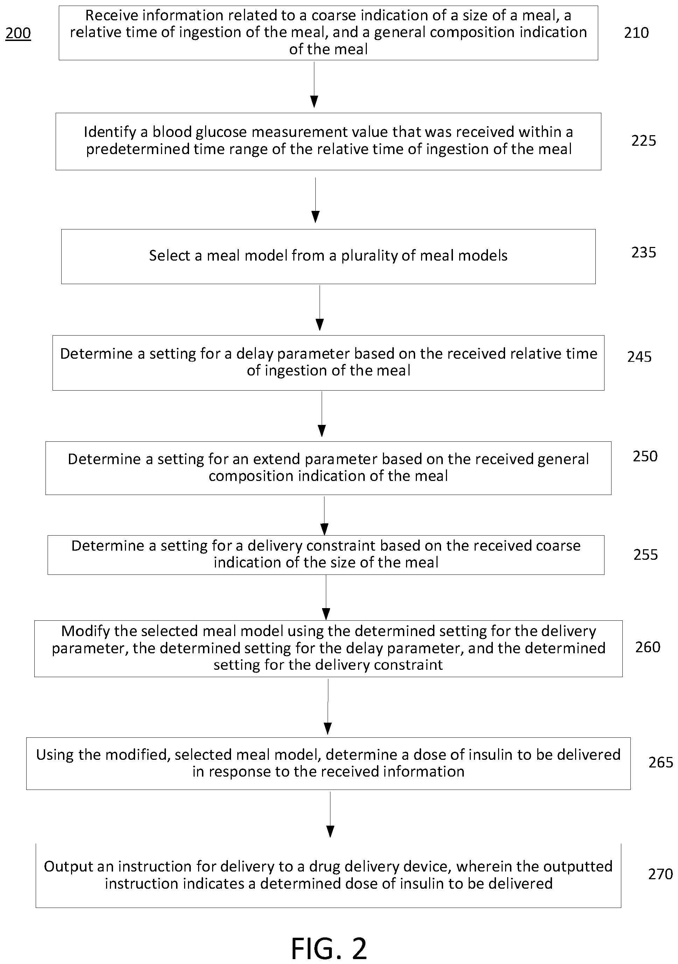

FIG. 2 illustrates another flowchart of an example process implemented by an artificial pancreas application using the techniques described in the example of FIG. 1.

In the example process 200, the AP application may be operable to receive information related to ingestion of a meal, wherein the information includes a coarse indication of a size of the meal, a relative time of ingestion of the meal, and a general composition indication of the meal (210). For example, the AP application may be operable to receive the information relate to ingestion of the meal from a user interface such as a graphical user interface, a keyboard, a microphone, a camera or the like. The AP application, in response to receiving the information, may identify a blood glucose measurement value that was received within a predetermined time range of the relative time of ingestion of the meal (225).

In the example, the AP application select a meal model from a number of meal models (235). To explain in more detail, a number of meal models may be stored in a memory accessible by the AP application. A meal model may be an algorithm having a number of parameters. For example, the AP application may monitor and develop known metrics related to the user's diabetes, such as their Hemoglobin A1c (HbA1c), a standard clinical method to assess the user's quality of glucose control over the previous 2-3 months, and % times in safe glycemic range, which is a measure to determine how much of their daily diabetes care the users' glucose concentrations remain in the approximately 70-180 mg/dL range that is between the hyperglycemic and hypoglycemic thresholds. The AP application may also specifically monitor and recommend the user's known clinical parameters related to diabetes, such as Total Daily Insulin (TDI), Insulin Onboard (IOB), Correction Factor, and Carbohydrate Ratio. The parameters may also be based on a number of inputs to the AP application that receive data related to a user associated with the AP application. The received data a historical profile of the user, a history of the user's insulin treatment, a history of the user's blood glucose measurement values and the like may be stored in a memory accessible by the AP application. The meal model used by the AP application may be a default algorithm when initially installed on a portable computing device but may develop into a customized algorithm after use by the user for a period of time. Alternatively, the meal model may be selected based on a blood glucose measurement that is identified as being received within a predetermined time range of the relative time of ingestion of the meal as indicated in the received information. In a further example, as the user continues to use the AP application, the AP application over time may be operable to develop customized parameter settings based performance of the meal model. In addition, there may be several default meal models that are specific to particular meals or times of day. For example, breakfast time meals may have a specific meal model, while dinner time meals may have a different but equally specific meal model. Other meal models may include a snack meal model, a lunch meal model, a rescue meal model, an exercise meal model or the like.

In the example of FIG. 2, the AP application may be further operable to determine a setting for a delay parameter based on the received relative time of ingestion of the meal (245). For example, the AP application may be operable to receive an input indicating the setting for the delay parameter and, based on the received input, may set the delay parameter in the selected meal model to a numeric value that corresponds to the received relative time of ingestion of the meal.

The AP application may be further operable to determine a setting for an extend parameter based on the received general composition indication of the meal. For example; using the received input indicating the general composition of the meal, the AP application may set the extend parameter to a numeric value based on the general composition of the meal (250).

At 255, the AP application may be operable to determine a setting for a delivery constraint based on the received coarse indication of the size of the meal. In an example, the AP application may receive, via a user interface or the like of the computing device, an input indicating the setting for the delivery constraint. Based on the received input, the AP application may set the delivery constraint to a numeric value. In a specific example, the AP application may determine the received input indicating the setting for the delivery constraint is an input indicating the ingested meal is a large meal. Based on the input indicating the ingested meal is a large meal, the AP application may be operable to set the delivery constraint to a very relaxed preset percentage above a maximum amount of insulin to be delivered in a day via automated delivery for basal compensation (i.e., basal coverage of total daily insulin (TDI), which is typically 50%). A very relaxed preset percentage above a maximum basal coverage of TDI may be between 40-50 percent, or the like. Alternatively, the AP application may determine the received input indicating the setting for the delivery constraint is an input indicating the ingested meal is a medium meal. Based on the ingested meal being a medium meal, the AP application may set the delivery constraint to a relaxed preset percentage above a maximum amount of insulin to be delivered (i.e., basal coverage of total daily insulin (TDI)). A relaxed preset percentage above a maximum basal coverage of TDI may be between 20-30 percent, or the like. In yet another alternative, the AP application may determine the received input indicating the setting for the delivery constraint is an input indicating the ingested meal is a small meal. Based on the input indicating the ingested meal is a small meal, the AP application may be operable to set the delivery constraint to a typical preset percentage above a maximum amount of insulin to be delivered (i.e., basal coverage of total daily insulin (TDI)). A typical preset percentage above a maximum basal coverage of TDI may be between 0-20 percent, or the like.

Upon setting of the respective parameters for extend, delay and delivery constraints, the AP application may modify the selected meal model using the determined setting for the delay parameter, the determined setting for the extend parameter, and the determined setting for the delivery constraint (260).

The model itself may include increased insulin delivery as a reaction to increasing blood glucose measurement values that are greater than normal with the knowledge the user had recently eaten. The magnitude of the reaction to the blood glucose measurement values that are allowable may be determined by the sub-model selected and known metrics such as Total Daily Insulin, Correction Factor, and Carbohydrate Ratio. The initial treatment target may also be adjusted to compensate for a larger amount of insulin onboard (IOB) during this period. A determination between an immediate bolus size and an extended bolus size and length may also be determined based on predictions may using the model. Other factors may also be considered such as, for example, whether the user participated in exercise and how recent was the exercise.

The AP application, using the modified, selected meal model, may be operable to determine a dose of insulin to be delivered in response to the received information (265). Upon determining a dose of insulin, the AP application may generate an instruction that indicates a determined dose of insulin to be delivered and, at 270, may output the instruction for delivery to a drug delivery device (shown in another example). The outputted instruction may indicate a determined dose of insulin to be delivered.

In some examples, the AP application may be operable to perform additional functions when selecting the meal model from the number of meal models. For example, the AP application may determine factors usable in limiting the number of meal models for selection from the number of meal models. For example, the AP application may use factors such as a time of day, calendar setting for a day in which the meal is being ingested, a closest match to a meal profile trajectory, user inputs (such as indications of meal composition), or the like. Using the factors, the AP application may be operable to identify meal models from the plurality of meal models that satisfy a greatest number of factors. The AP application may eliminate all meal models from the plurality of meal models except the identified meal models and may choose one of the identified meal models for selection based on a confidence level associated with each of the identified meal models.

The confidence level may be probability score of how correct the AP application is in the identification of the meal being ingested. The confidence score may be based on a number of factors, such as, for example, how accurate the user is responding to the prompts at 120, 140 and 160 of FIG. 1. The user's accuracy may, for example, be determined by evaluating blood glucose measurement values received subsequent to ingestions of the meal or based on the fact the user is not requesting another bolus after receiving a meal-related bolus. If the user is accurate a high percentage of the time based on a user history, the AP application confidence level in the selected meal model may be high. Conversely, if the user is frequently inaccurate, other factors, such as past meal history (i.e., eating large dinner meals at around 6:00 PM every weekday or the like), and corresponding blood glucose measurement values, may be used to determine the confidence level of the respective meal models.

In a further example, prior to modifying the selected meal model, the AP application may receive blood glucose measurement values over a period of time extending from before until after the receipt of the relative time of ingestion of the meal. In the further example, the AP application may be operable to determine a trajectory of the received blood glucose measurement values and compare the determined trajectory to known blood glucose measurement value trajectories related to meals of different compositions. Based on a result of the comparison, the AP application may determine the user accurately described the meal and confirm that the received input indicating the setting for the extend parameter is correct. The AP application may utilize the received input in the determination of the extend parameter based on the confirmation.

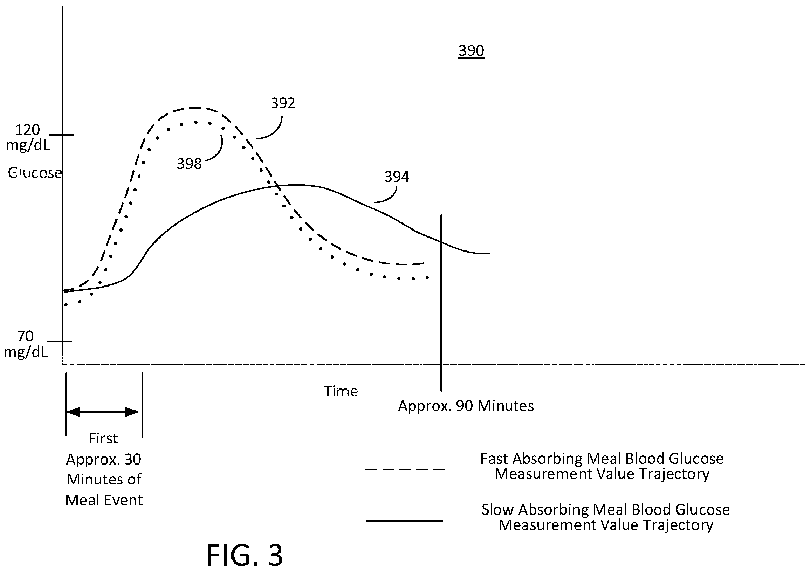

FIG. 3 is graph illustrating blood glucose profiles with respect to high absorbing food and slow absorbing food. In another example, the rate of change of blood glucose measurement values following ingestion of a meal may be utilized to assess a relative composition of meals - for example, blood glucose measurement values with high rates of change may be considered to be the result of a meal with faster absorbing carbohydrates, whereas a relatively flat glucose trend may be considered high fat meals.

Processed foods are quickly absorbed by the body due to the high sugar content of the processed foods so meals containing processed foods, in particular, the carbohydrates in processed foods are considered fast absorbing because of the high sugar content. High fiber and high protein meals are more slowly absorbed by the body. In the example, the graph 390 illustrates a y-axis representing approximate blood glucose measurement values in units of mg/dL and the x-axis is approximate times in minutes. A trajectory of the blood glucose measurement values in response to a fast absorbing (possibly low fat) meal (as represented by trajectory 392) usually rises rapidly (as shown by the dashed line), whereas a trajectory of the blood glucose measurement values in response to a slow absorbing (due to possible high fat content) meal (as represented by trajectory 394) usually rises slowly (as shown by the solid line). The steeper the rise of the trajectory of the blood glucose measurement values the greater the rate of change of the user's blood glucose measurement values. For example, the trajectory 392 rises more rapidly than the trajectory 394.

In FIG. 3, the time bracket below the time axis may, for example, represents the first approximately 30 minutes of time after the ingestion of a meal. The algorithm or AP application may be operable to analyze, for example, the first XX minutes of a blood glucose measurement value trajectory following meal ingestion and determining whether the of blood glucose measurement value is rising rapidly or slowly. In an operational example, an algorithm or AP application may receive blood glucose measurements from a continuous blood glucose monitor or the like. The algorithm or AP application may be operable to receive an initial blood glucose measurement value from a continuous blood glucose monitor or other blood glucose measurement device at a time indicated as an approximate time of ingestion of a meal, for example, the approximate time may be based on a calendar application data, a user's direct input to a meal button, a combination of data such as location data and built-in sensor data, such as a camera image of a restaurant or accelerometer data indicating standing, sitting or casual interaction. The algorithm or AP application may also receive subsequent blood glucose measurement values. The algorithm or AP application may be further operable to analyze the initial and subsequent blood glucose measurement values to determine whether a rate of change of the blood glucose measurement values more closely matches the fast absorbing meal blood glucose measurement value trajectory 392 or a slow absorbing meal blood glucose measurement value trajectory 394. The closeness of the match between the respective rates of change may be determined using, for example, a curve-fitting algorithm or other form of function. For example, the algorithm or AP application may be operable to receive the initial and subsequent blood glucose measurement values and compare the initial and subsequent blood glucose measurement values to reference blood glucose measurement values stored, for example, in memory. Based on the result of the comparison, the algorithm or AP application may determine whether the ingested meal is slow-absorbing or fast absorbing and generate an appropriate response for modifying an insulin treatment plan of the user.

In the specific example of the process 200, the respective trajectories may be used to in the determination of a setting for the extend parameter. For example, during the process 200, the AP application may receive blood glucose measurement values (shown as black dots in the curve 398) over a period of time extending from before until after the receipt of the relative time of ingestion of the meal. The AP application may be operable to determine a trajectory of the received blood glucose measurement values shown as 398. The AP application may compare the determined trajectory to a fast absorbing meal blood glucose measurement value trajectory. Based on a result of the comparison, the AP application may quantify a fast-absorbing similarity value between the determined trajectory and the fast absorbing meal blood glucose measurement value trajectory. The AP application may be further operable to compare the determined trajectory to a slow absorbing meal blood glucose measurement value trajectory and, based on a result of the comparison, may quantify a slow-absorbing similarity between the determined trajectory and the slow absorbing meal blood glucose measurement value trajectory. The AP application may be operable to determine which of the quantified fast-absorbing similarity and the quantified slow-absorbing similarity has a highest similarity value. Based on the highest similarity value, the AP application may determine whether the fast absorbing meal blood glucose measurement value trajectory or the slow absorbing meal blood glucose measurement value trajectory is most similar to the determined trajectory. As a result of the determination of the most similar to the determined trajectory, the AP application may be operable to use the result of the determination (i.e. which of the fast absorbing meal blood glucose measurement value trajectory or the slow absorbing meal blood glucose measurement value trajectory is most similar) based on the highest similarity value in the further determination of the extend parameter. For example, the determination of the extend parameter may be a fixed value that is an ON or OFF determination depending upon which of the quantified fast-absorbing similarity or the quantified slow-absorbing similarity has a highest similarity value.

In the examples of FIGs. 1-3, the example processes may be implemented by programming code, such as an AP application or an algorithm, that is executed by a processor. The AP application or algorithm when executed by a processor may utilize inputs and calculations as described with respect to the foregoing examples.

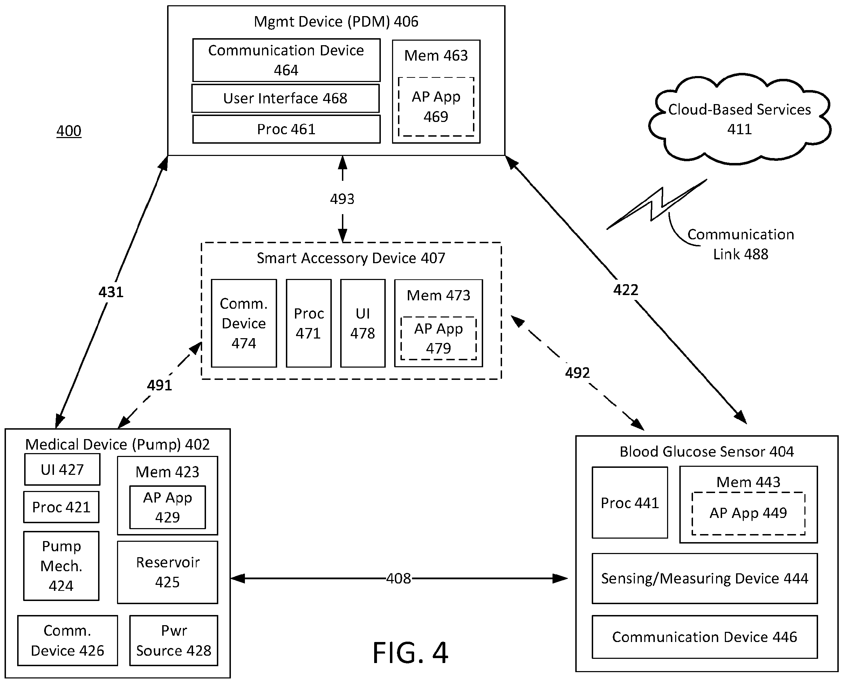

It may be helpful to discuss an example of a drug delivery system that may implement the process example of FIGs. 1-3. FIG. 4 illustrates an example of a drug delivery system 400.

The drug delivery system 400 may be operable to implement the process examples illustrated in FIGs. 1-3 by executing an AP application or algorithm that includes functionality related to receiving information related to ingestion of a meal. The information may include a coarse indication of a size of the meal, a relative time of ingestion of the meal, and a general composition indication of the meal. A blood glucose measurement value may be received within a predetermined time range of a relative time of ingestion of the meal. Settings for a delay and an extend parameter and a delivery constraint may be determined. A meal model may be modified using the determined settings for the delay parameter, the extend parameter, and the delivery constraint. The modified meal model may be used to determine a dose of insulin to be delivered in response to the received information. An instruction indicates a determined dose of insulin to be delivered may be output for delivery to a drug delivery device.

The drug delivery system 400 may be an automated drug delivery system that may include a medical device (pump) 402 (also referred to as "a drug delivery device" or "a wearable drug delivery device"), a blood glucose sensor 404 (also referred to as "a continuous glucose monitor" or "a blood glucose measurement device"), and a management device (PDM) 406. The system 400, in an example, may also include a smart accessory device 407, which may be operable to communicate with the other components of system 400 either via a wired or wireless communication link, such as 491, 492 or 493.

In an example, the medical device 402 may be attached to the body of a user, such as a patient or diabetic, and may deliver any therapeutic agent, including any drug or medicine, such as insulin, morphine or the like, to the user. The medical device 402 may, for example, be a wearable device worn by the user. For example, the medical device 402 may be directly coupled to a user (e.g., directly attached to a body part and/or skin of the user via an adhesive or the like). In an example, a surface of the medical device 402 may include an adhesive (not shown) to facilitate attachment to a user.

The medical device 402 may include a number of components to facilitate automated delivery of a drug (also referred to as a therapeutic agent) to the user. The medical device 402 may be operable to store the drug (i.e., insulin) and to provide the drug to the user. The medical device 402 is often referred to as a pump, or an insulin pump, in reference to the operation of expelling insulin from the reservoir 425 for delivery to the user. While the examples refer to the reservoir 425 storing insulin, the reservoir 425 may be operable to store other drugs or therapeutic agents, such as morphine or the like, that are suitable for automated delivery.

In various examples, the medical device 402 may be an automated, wearable drug delivery device. For example, the medical device 402 may include a reservoir 425 for storing the drug (such as insulin), a needle or cannula (not shown) for delivering the drug into the body of the user (which may be done subcutaneously, intraperitoneally, or intravenously), and a pump mechanism (mech.) 424, or other drive mechanism, for transferring the drug from the reservoir 425, through a needle or cannula (not shown), and into the user. The pump mechanism 424 may be fluidly coupled to reservoir 425, and communicatively coupled to the medical device processor 421. The medical device 402 may also include a power source 428, such as a battery, a piezoelectric device, or the like, for supplying electrical power to the pump mechanism 424 and/or other components (such as the processor 421, memory 423, and the communication device 426) of the medical device 402. Although not shown, an electrical power supply for supplying electrical power may similarly be included in each of the sensor 404, the smart accessory device 407 and the management device (PDM) 406.

The blood glucose sensor 404 may be a device communicatively coupled to the processor 461 or 421 and may be operable to measure a blood glucose value at a predetermined time intervals, such as every 5 minutes, or the like. The blood glucose sensor 404 may provide a number of blood glucose measurement values to the AP applications operating on the respective devices (e.g., 429, 449, 469, or 479) at predetermined time intervals, such as every 5 minutes or the like.

The medical device 402 may provide the insulin stored in reservoir 425 to the user based on information (e.g., blood glucose measurement values, predicted future blood glucose measurements, evaluations based on a user request for a bolus, an user interaction with PDM 406, medical device 402, sensor 404 or smart accessory device 407), evaluations of missing blood glucose measurements and the other information provided by the sensor 404, smart accessory device 407, and/or the management device (PDM) 406. For example, the medical device 402 may receive instruction outputted by the AP application 469 (479 or 449) that indicate a determined dose of insulin to be delivered and in response to the received instruction may deliver the determined dose of insulin to the user.

In an example, the medical device 402 may contain analog and/or digital circuitry that may be implemented as a processor 421 (or controller) for controlling the delivery of the drug or therapeutic agent. The circuitry used to implement the processor 421 may include discrete, specialized logic and/or components, an application-specific integrated circuit, a microcontroller or processor that executes software instructions, firmware, programming instructions or programming code (enabling, for example, the artificial pancreas application (AP App) 429 as well as the process examples of FIGs. 1-3) stored in memory 423, or any combination thereof. For example, the processor 421 may execute a control algorithm, such as an artificial pancreas application 429, and other programming code that may make the processor 421 operable to cause the pump to deliver doses of the drug or therapeutic agent to a user at predetermined intervals or as needed to bring blood glucose measurement values to a target blood glucose value. In an example, the AP application (App) 429 may include programming code that is operable upon execution by the processor 421 to provide the example processes for adjusting or modifying duration of insulin action settings, confidence values, insulin delivery settings, storing blood glucose measurement values in memory, or the like as described with reference to FIGs. 1-3. The size and/or timing of the doses may be programmed, for example, into an artificial pancreas application 429 by the user or by a third party (such as a health care provider, medical device manufacturer, or the like) using a wired or wireless communication link, such as 431, between the medical device 402 and a management device 406 or other device, such as a computing device at a healthcare provider facility. In an example, the pump or medical device 402 is communicatively coupled to the processor 461 of the management device via the wireless communication link 431 or via a wireless communication link, such as 491 from smart accessory device 407 or 408 from the sensor 404. The pump mechanism 424 of the medical device 402 may be operable to receive an actuation signal from the processor 461, and in response to receiving a command signal or actuation signal, expel insulin from the reservoir 425 based on the evaluations and process steps performed in the process examples of FIGs. 1-3.

In an operational example, the AP application 469 may be executing in the management device 406 and control delivery of insulin. For example, the AP application 469 may be operable to determine timing of an insulin dose and may output a command signal to the medical device 402 that actuates the pump mechanism 424 to deliver insulin dose based on the evaluations and process steps performed in the process examples of FIGs. 1-3.

The other devices in the system 400, such as management device 406, smart accessory device 407 and sensor 404, may also be operable to perform various functions including controlling the medical device 402. For example, the management device 406 may include a communication device 464, a processor 461, and a management device memory 463. The management device memory 463 may store an instance of the AP application 469 that includes programming code, that when executed by the processor 461 provides the process examples described with reference to the examples of FIGs. 1-3. The management device memory 463 may also store programming code for providing the process examples described with reference to the examples of FIGs. 1-3.

The smart accessory device 407 may be, for example, an Apple Watche, other wearable smart device, including eyeglasses, provided by other manufacturers, a global positioning system-enabled wearable, a wearable fitness device, smart clothing, or the like. Similar to the management device 406, the smart accessory device 407 may also be operable to perform various functions including controlling the medical device 402. For example, the smart accessory device 407 may include a communication device 474, a processor 471, and a memory 473. The memory 473 may store an instance of the AP application 479 that includes programming code for providing the process examples described with reference to the examples of FIGs. 1 and 2. The memory 473 may also as store programming code and be operable to store data related to the AP application 479. The sensor 404 of system 400 may be a continuous glucose monitor (CGM) as described above, that may include a processor 441, a memory 443, a sensing or measuring device 444, and a communication device 346. The memory 443 may, for example, store an instance of an AP application 449 as well as other programming code and be operable to store data related to the AP application 449 and process examples described with reference to FIGs. 1-3. The AP application 449 may also include programming code for providing the process examples described with reference to the examples of FIGs. 1-3.

Instructions for determining the delivery of the drug or therapeutic agent (e.g., as a bolus dosage) to the user (e.g., the size and/or timing of any doses of the drug or therapeutic agent) may originate locally by the medical device 402 or may originate remotely and be provided to the medical device 402. In an example of a local determination of drug or therapeutic agent delivery, programming instructions, such as an instance of the artificial pancreas application 429, stored in the memory 423 that is coupled to the medical device 402 may be used to make determinations by the medical device 402. In addition, the medical device 402 may be operable to communicate with the cloud-based services 411 via the communication device 426 and the communication link 488.

Alternatively, the remote instructions may be provided to the medical device 402 over a wired or wireless communication link (such as 431) by the management device (PDM) 406, which has a processor 461 that executes an instance of the artificial pancreas application 469, or the smart accessory device 407 (via communication link 491), which has a processor 471 that executes an instance of the artificial pancreas application 469 as well as other programming code for controlling various devices, such as the medical device 402, smart accessory device 407 and/or sensor 404. The medical device 402 may execute any received instructions (originating internally or from the management device 406) for the delivery of the drug or therapeutic agent to the user. In this way, the delivery of the drug or therapeutic agent to a user may be automated.

In various examples, the medical device 402 may communicate via a wireless communication link 431 with the management device 406. The management device 406 may be an electronic device such as, for example, a smart phone, a tablet, a dedicated diabetes therapy management device, or the like. The management device 406 may be a wearable wireless accessory device. The wireless communication links 408, 431, 422, 491, 492 and 493 may be any type of wireless communication link provided by any known wireless standard. As an example, the wireless communication links 408, 431, 422, 491, 492 and 493 may enable communications between the medical device 402, the management device 406 and sensor 404 based on, for example, Bluetooth^{®}, Wi-Fi^{®}, a near-field communication standard, a cellular standard, or any other wireless optical or radio-frequency protocol.

The sensor 404 may be a glucose sensor operable to measure blood glucose and output a blood glucose value or data that is representative of a blood glucose value. For example, the sensor 404 may be a glucose monitor or a continuous glucose monitor (CGM). The sensor 404 may include a processor 441, a memory 443, a sensing/measuring device 444, and communication device 446. The communication device 446 of sensor 404 may include one or more sensing elements, an electronic transmitter, receiver, and/or transceiver for communicating with the management device 406 over a wireless communication link 422 or with medical device 402 over the link 408. The sensing/measuring device 444 may include one or more sensing elements, such as a glucose measurement, heart rate monitor, or the like. The processor 441 may include discrete, specialized logic and/or components, an application-specific integrated circuit, a microcontroller or processor that executes software instructions, firmware, programming instructions stored in memory (such as memory 443), or any combination thereof. For example, the memory 443 may store an instance of an AP application 449 that is executable by the processor 441.

Although the sensor 404 is depicted as separate from the medical device 402, in various examples, the sensor 404 and medical device 402 may be incorporated into the same unit. That is, in various examples, the sensor 404 may be a part of the medical device 402 and contained within the same housing of the medical device 402 (e.g., the sensor 404 may be positioned within or embedded within the medical device 402). Glucose monitoring data (e.g., measured blood glucose values) determined by the sensor 404 may be provided to the medical device 402, smart accessory device 407 and/or the management device 406 and may be used to perform the functions and deliver doses of insulin for automated delivery of insulin by the medical device 402 as described with reference to the examples of FIGs. 1-3.

The sensor 404 may also be coupled to the user by, for example, adhesive or the like and may provide information or data on one or more medical conditions and/or physical attributes of the user. The information or data provided by the sensor 404 may be used to adjust drug delivery operations of the medical device 402.

In an example, the management device 406 may be a computing device operable to manage a personal diabetes treatment plan via an AP application or an algorithm. The management device 406 may be used to program or adjust operation of the medical device 402 and/or the sensor 404. The management device 406 may be any portable electronic, computing device including, for example, a dedicated controller, such as processor 461, a smartphone, or a tablet. In an example, the management device (PDM) 406 may include a processor 461, a management device management device memory 463, and a communication device 464. The management device 406 may contain analog and/or digital circuitry that may be implemented as a processor 461 (or controller) for executing processes to manage a user's blood glucose levels and for controlling the delivery of the drug or therapeutic agent to the user. The processor 461 may also be operable to execute programming code stored in the management device management device memory 463. For example, the management device management device memory 463 may be operable to store an artificial pancreas (AP) application 469 that may be executed by the processor 461. The processor 461 may when executing the artificial pancreas application 469 may be operable to perform various functions, such as those described with respect to the examples in FIGs. 1-3. The communication device 464 may be a receiver, a transmitter, or a transceiver that operates according to one or more radio-frequency protocols. For example, the communication device 464 may include a cellular transceiver and a Bluetooth transceiver that enables the management device 406 to communicate with a data network via the cellular transceiver and with the sensor 404 and the medical device 402. The respective transceivers of communication device 464 may be operable to transmit signals containing information useable by or generated by the AP application or the like. The communication devices 426, 446 and 476 of respective medical device 402, sensor 404 and smart accessory device 407 may also be operable to transmit signals containing information useable by or generated by the AP application or the like.

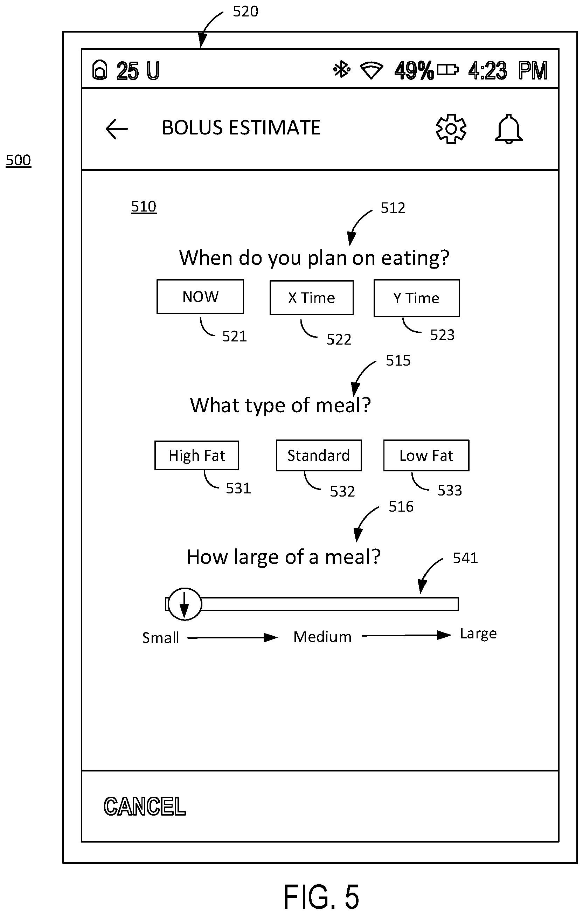

The medical device 402 may communicate with the sensor 404 over a wireless communication link 408 and may communicate with the management device 406 over a wireless communication link 431. The sensor 404 and the management device 406 may communicate over a wireless communication link 422. The smart accessory device 407, when present, may communicate with the medical device 402, the sensor 404 and the management device 406 over wireless communication links 491, 492 and 493, respectively. The wireless communication links 408, 431, 422, 491, 492 and 493 may be any type of wireless communication link operating using known wireless standards or proprietary standards. As an example, the wireless communication links 408, 431, 422, 491, 492 and 493 may provide communication links based on Bluetooth^{®}, Wi-Fi, a near-field communication standard, a cellular standard, or any other wireless protocol via the respective communication devices 426, 446 and 464. In some examples, the medical device 402 and/or the management device 406 may include a user interface 427, 478 and 468, respectively, such as a keypad, a touchscreen display, levers, buttons, a microphone, a speaker, a display, or the like, that is operable to allow a user to enter information and allow the management device to output information for presentation to the user. One of many examples of a user interface, such as 427, 478 or 468, suitable for use in implementing the techniques described herein is described below with reference to FIG. 5.

In various examples, the drug delivery system 400 may implement the artificial pancreas (AP) algorithm (and/or provide AP functionality) to govern or control automated delivery of insulin to a user (e.g., to maintain euglycemia - a normal level of glucose in the blood). The AP application may be implemented by the medical device 402 and/or the sensor 404. The AP application, in addition to the processes described with reference to the examples of FIGs. 1-3, may be used to determine the times and dosages of insulin delivery. In various examples, the AP application may determine the times and dosages for delivery based on information known about the user, such as the user's sex, age, weight, or height, and/or on information gathered about a physical attribute or condition of the user (e.g., from the sensor 404). For example, the AP application may determine an appropriate delivery of insulin based on glucose level monitoring of the user through the sensor 404. The AP application may also allow the user to adjust insulin delivery. For example, the AP application may allow the user to issue (e.g., via an input) commands to the medical device 402, such as a command to deliver an insulin bolus. In some examples, different functions of the AP application may be distributed among two or more of the management device 406, the medical device (pump) 402 or the sensor 404. In other examples, the different functions of the AP application may be performed by one device, such the management device 406, the medical device (pump) 402 or the sensor 404.

In an example of a hardware implementation of a real-time meal model adjustment, the example processes, such as those described with reference to FIGs. 1-3 above may be implemented on a blood glucose sensor 404. The blood glucose sensor 404 may, for example, be a continuous blood glucose monitor (CGM) that may be operable via AP application 449 to receive insulin delivery information from a personal diabetes management device 406.

As described herein, the drug delivery system 400 or any component thereof, such as the medical device may be considered to provide AP functionality or to implement an AP application. Accordingly, references to the AP application (e.g., functionality, operations, or capabilities thereof) are made for convenience and may refer to and/or include operations and/or functionalities of the drug delivery system 400 or any constituent component thereof (e.g., the medical device 402 and/or the management device 406). The drug delivery system 400 - for example, as an insulin delivery system implementing an AP application - may be considered to be a drug delivery system or an AP application-based delivery system that uses sensor inputs (e.g., data collected by the sensor 404).

In an example, one or more of the devices, 402, 404, 406 or 407 may be operable to communicate via a wireless communication link 488 with cloud-based services 411. The cloud-based services 411 may utilize servers and data storage (not shown). The communication link 488 may be a cellular link, a Wi-Fi link, a Bluetooth link, or a combination thereof, that is established between the respective devices 402, 404, 406 or 407 of system 400. The data storage provided by the cloud-based services 411 may store anonymized data, such as user weight, blood glucose measurements, age, meal carbohydrate information, or the like. In addition, the cloud-based services 411 may process the anonymized data from multiple users to provide generalized information related to the various parameters used by the AP application. For example, an age-based general target blood glucose value may be derived from the anonymized data, which may be helpful in selection of a meal model from the number of meal models available for selection while using a system such as 400. The cloud-based services 411 may also provide processing services for the system 400, such as performing the process 100 in the example of FIG. 1 or additional processes, such as those described with reference to FIGs. 2 and 3.

In an example, the device 402 includes a communication device 464, which as described above may be a receiver, a transmitter, or a transceiver that operates according to one or more radio-frequency protocols, such as Bluetooth, Wi-Fi, a near-field communication standard, a cellular standard, that may enable the respective device to communicate with the cloud-based services 411. For example, outputs from the sensor 404 or the medical device (pump) 402 may be transmitted to the cloud-based services 411 for storage or processing via the transceivers of communication device 464. Similarly, medical device 402, management device 406 and sensor 404 may be operable to communicate with the cloud-based services 411 via the communication link 488.

In an example, the respective receiver or transceiver of each respective device, 402, 406 or 407, may be operable to receive signals containing respective blood glucose measurement values of the number of blood glucose measurement values that may be transmitted by the sensor 404. The respective processor of each respective device 402, 406 or 407 may be operable to store each of the respective blood glucose measurement values in a respective memory, such as 423, 463 or 473. The respective blood glucose measurement values may be stored as data related to the artificial pancreas algorithm, such as 429, 449, 469 or 479. In a further example, the AP application operating on any of the management device 406, the smart accessory device 407, or sensor 404 may be operable to transmit, via a transceiver implemented by a respective communication device, 464, 474, 446, a control signal for receipt by a medical device. In the example, the control signal may indicate an amount of insulin to be expelled by the medical device 402.

Various operational scenarios and examples of processes performed by the system 400 are described herein. For example, the system 400 may be operable to implement the process examples of FIG. 1-3 and user interface and graphical user interface example of FIG. 5, which is described below.

The techniques described herein for providing functionality to may be implemented using a number of different approaches. For example, the system 400 or any component thereof may be implemented in hardware, software, or any combination thereof. Software related implementations of the techniques described herein may include, but are not limited to, firmware, application specific software, or any other type of computer readable instructions that may be executed by one or more processors. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

FIG. 5 illustrates an example of a graphical user interface presented on a user interface as described with reference to the examples of FIGs. 1-4.

The user interface 520 may be presented on a computing device such as smartphone, a personal diabetes management device, a tablet or a wearable electronic device, such as a smartwatch or the like. In addition to providing other information related to diabetes management, the user interface 520 may also include a graphical user interface 510 that includes prompts for information useful in calculating a bolus dosage. The user interface 520 may be for example a touchscreen display that is operable in response to commands from a computing device processor (shown in other examples) to present prompts and input icons. For example, the prompts, such as 512, 515 and 516, may present the queries described with reference to FIG. 1 above. For example, the prompt 512 enables an input indicating a relative time of ingestion of the meal. In the example, the prompt 512 may be "when do you plan on eating?" and the input icons (521-523) may present the responses of "NOW," (521) "X time" (522) or "Y time" (523). The prompt 515 may enable an input indicating a general composition indication of the meal. For example, the prompt 515 may be, for example, the query "What type of meal?" The input icons 531, 532 and 533 may, respectively, be "High Fat," "Standard" and "Low Fat" or the like may be presented. A further query may be a request for an indication of the size of the meal. For example, the prompt may be a query of "how large of a meal?" The input icon may be replaced with a slider input 541 that indicates a coarse indication of a size of a meal by providing a range of meal size indications from "Small" to "Large" with "Medium" being in between Small and Large. The illustrated example of the user interface 520 and the graphical user interface 510 are shown for purposes of illustration and are not shown to limit the user interface 520 or graphical user interface 510 to these specific examples. Of course, the user interface 520 may present hard buttons as opposed to the soft buttons of the input icons 521-523, for example. Other alternative input mechanisms and devices may also be considered as described herein.

Alternatively, the graphical user interface 510 may present a simple meal button (e.g., meal indicator and the time of day, if the user is consistent with the size and types of meals eaten) that may associate different meal models within the algorithm or AP application to improve post prandial control while reducing user burden.

In addition, or alternatively, while the examples may have been described with reference to a closed loop algorithmic implementation of an AP application or an algorithm, variations of the disclosed examples may be implemented to enable open loop use. The open loop implementations allow for use of different modalities of delivery of insulin such as smart pen, syringe or the like, with the trigger of the initiation of insulin deliveries being the user, rather than an automated algorithm. For example, the disclosed AP application and algorithms may be operable to perform various functions related to open loop operations, such as outputting an instruction including a determined dose of insulin to be delivered to a user that presents via a user interface the determined dose of insulin. Other open-loop actions may also be implemented by adjusting user settings or the like in an AP application or algorithm.

Some examples of the disclosed device may be implemented, for example, using a storage medium, a computer-readable medium, or an article of manufacture which may store an instruction or a set of instructions that, if executed by a machine (i.e., processor or microcontroller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Program aspects of the technology may be thought of as "products" or "articles of manufacture" typically in the form of executable code and/or associated data that is carried on or embodied in a type of machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of example examples has been presented for the purposes of illustration and description. It is not intended to be exhaustive or to limit the present disclosure to the precise forms disclosed. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

## Claims

1. A non-transitory computer readable medium embodied with programming code executable by a processor, and the processor when executing the programming code is operable to perform functions, including functions to:
receive (210) information related to ingestion of a meal, wherein the information includes a coarse indication of a size of the meal, a relative time of ingestion of the meal, and a general composition indication of the meal;
identify (225) a blood glucose measurement value that was received within a predetermined time range of the relative time of ingestion of the meal;
select (235) a meal model from a plurality of meal models;
determine (245) a setting for a delay parameter based on the received relative time of ingestion of the meal;
determine (250) a setting for an extend parameter based on the received general composition indication of the meal;
determine (255) a setting for a delivery constraint based on the received coarse indication of the size of the meal;
modify (260) the selected meal model using the determined setting for the delay parameter, the determined setting for the extend parameter, and the determined setting for the delivery constraint;
using (265) the modified, selected meal model, determine a dose of insulin to be delivered in response to the received information; and
output (270) an instruction for delivery to a drug delivery device, wherein the outputted instruction indicates a determined dose of insulin to be delivered.

2. The non-transitory computer readable medium of claim 1, wherein the processor when executing the programming code is operable to perform further functions, including functions to:
receive an input indicating the setting for the delay parameter; and
based on the received input, set the delay parameter to a numeric value.

3. The non-transitory computer readable medium of claim 1, wherein the processor when executing the programming code is operable to perform further functions, including functions to:
receive an input indicating the setting for the extend parameter; and
based on the received input, set the extend parameter to a numeric value.

4. The non-transitory computer readable medium of claim 3, wherein the processor when executing the programming code is operable to perform further functions, including functions, when receiving the input indicating the setting for the extend parameter, to:
receive blood glucose measurement values over a period of time extending from before until after receipt of the relative time of ingestion of the meal;
determine a trajectory (398) of the received blood glucose measurement values;
compare the determined trajectory (398) to known blood glucose measurement value trajectories related to meals of different compositions;
based on a result of the comparison, determine the general meal composition indication was accurate;
confirm that the received input indicating the setting for the extend parameter is correct; and
based on the confirmation, utilize the received input in the determination of the extend parameter, wherein the extend parameter is a further time delay in addition to the delay parameter.

5. The non-transitory computer readable medium of claim 1, wherein the processor when executing the programming code is operable, when determining the setting for the delivery constraint, to perform further functions, including functions to:
receive an input indicating the setting for the delivery constraint; and
based on the received input, set the delivery constraint to a numeric value.

6. The non-transitory computer readable medium of claim 5, wherein the processor when executing the programming code is operable to perform further functions, including functions to:
determine the received input indicating the setting for the delivery constraint is an input indicating an ingested meal is a large meal; and
set the delivery constraint to a very relaxed preset percentage above a maximum amount of insulin to be delivered.

7. The non-transitory computer readable medium of claim 5, wherein the processor when executing the programming code is operable to perform further functions, including functions to:
determine the received input indicating the setting for the delivery constraint is an input indicating an ingested meal is a medium meal; and
set the delivery constraint to a relaxed preset percentage above a maximum amount of insulin to be delivered.

8. The non-transitory computer readable medium of claim 5, wherein the processor when executing the programming code is operable to perform further functions, including functions to:
determine the received input indicating the setting for the delivery constraint is an input indicating an ingested meal is a small meal; and
set the delivery constraint to a typical preset percentage above a maximum amount of insulin to be delivered.

9. The non-transitory computer readable medium of claim 1, wherein the processor when executing the programming code is operable to perform further functions, including functions, when selecting the meal model from the plurality of meal models, to:
determine factors usable in limiting a number of meal models for selection from the plurality of meal models, wherein the factors include a time of day, calendar setting for a day in which the meal is being ingested, a closest match to a meal profile trajectory, or user inputs;
identify meal models from the plurality of meal models that satisfy a greatest number of factors;
eliminate all meal models from the plurality of meal models except for the identified meal models; and
choose one of the identified meal models for selection based on a confidence level associated with each of the identified meal models.

10. The non-transitory computer readable medium of claim 1, wherein the processor when executing the programming code is operable to perform further functions, including functions to:
receive blood glucose measurement values over a period of time extending from before until after receipt of the relative time of ingestion of the meal;
determine a trajectory (398) of the received blood glucose measurement values;
compare the determined trajectory (398) to a fast absorbing meal blood glucose measurement value trajectory;
quantify a fast-absorbing similarity value between the determined trajectory and the fast absorbing meal blood glucose measurement value trajectory;
compare the determined trajectory to a slow absorbing meal blood glucose measurement value trajectory;
quantify a slow-absorbing similarity a similarity between the determined trajectory and the slow absorbing meal blood glucose measurement value trajectory;
based on a highest similarity value, determine the extend parameter, wherein the extend parameter is a further time delay in addition to the delay parameter.

11. A device, comprising:
a processor (461);
a memory (463) storing programming code, an artificial pancreas application (469), and operable to store data related to the artificial pancreas application, wherein the programming code and the artificial pancreas application are executable by the processor (461); and
wherein the processor when executing the artificial pancreas application is operable to control delivery of insulin, and to perform functions, including functions to:
receive (210) information related to ingestion of a meal, wherein the information includes a coarse indication of a size of the meal, a relative time of ingestion of the meal, and a general composition indication of the meal;
identify (225) a blood glucose measurement value that was received within a predetermined time range of the relative time of ingestion of the meal;
select (235) a meal model from a plurality of meal models based on the identified blood glucose measurement value;
determine (245) a setting for a delay parameter based on the received relative time of ingestion of the meal;
determine (250) a setting for an extend parameter based on the received general composition indication of the meal;
determine (255) a setting for a delivery constraint based on the received coarse indication of the size of the meal;
modify (260) the selected meal model using the determined setting for the delay parameter, the determined setting for the extend parameter, and the determined setting for the delivery constraint;
using (265) the modified, selected meal model, determine a dose of insulin to be delivered in response to the received information; and
output (270) an instruction for delivery to a drug delivery device, wherein the outputted instruction indicates a determined dose of insulin to be delivered.

12. The device of claim 11, further comprising:
a transceiver operable to receive signals from and transmit signals to a blood glucose sensor (404),
wherein the transceiver is operable to:
receive a plurality of blood glucose measurement values, wherein respective blood glucose measurement values are received at respective predetermined time intervals; and
provide the measured blood glucose values to the processor (461) and the artificial pancreas application (469).

13. The device of claim 11, further comprising:
a transceiver operable to receive signals from and transmit signals to a drug delivery device (402),
wherein the transceiver is operable to:
forward the instruction to the drug delivery device (402) instructing delivery of the determined dose of insulin.

## Patentansprüche

1. Nichtflüchtiges computerlesbares Medium, auf dem durch einen Prozessor ausführbarer Programmcode vorliegt, wobei der Prozessor bei Ausführung des Programmcodes dazu betreibbar ist, Funktionen auszuführen, darunter Funktionen für Folgendes:
Empfangen (210) von Informationen bezüglich der Einnahme einer Mahlzeit, wobei die Informationen eine grobe Angabe einer Größe der Mahlzeit, eine relative Einnahmezeit der Mahlzeit und eine Angabe zur allgemeinen Zusammensetzung der Mahlzeit beinhaltet;
Identifizieren (225) eines Blutzuckermesswertes, der innerhalb eines vorbestimmten Zeitbereichs der relativen Einnahmezeit der Mahlzeit empfangen wurde;
Auswählen (235) eines Mahlzeitmodells aus einer Mehrzahl von Mahlzeitmodellen;
Bestimmen (245) einer Einstellung für einen Verzögerungsparameter basierend auf der empfangenen relativen Einnahmezeit der Mahlzeit;
Bestimmen (250) einer Einstellung für einen Erweiterungsparameter basierend auf der empfangenen Angabe zur allgemeinen Zusammensetzung der Mahlzeit;
Bestimmen (255) einer Einstellung für eine Abgabebeschränkung basierend auf der empfangenen groben Angabe der Größe der Mahlzeit;
Modifizieren (260) des ausgewählten Mahlzeitmodells unter Verwendung der bestimmten Einstellung für den Verzögerungsparameter, der bestimmten Einstellung für den Erweiterungsparameter und der bestimmten Einstellung für die Abgabebeschränkung;
Verwenden (265) des modifizierten, ausgewählten Mahlzeitmodells, Bestimmen einer abzugebenden Insulindosis in Reaktion auf die empfangenen Informationen; und
Ausgeben (270) einer Abgabeanweisung an eine Medikamentenabgabevorrichtung, wobei die ausgegebene Anweisung eine bestimmte abzugebende Insulindosis angibt.

2. Nichtflüchtiges computerlesbares Medium nach Anspruch 1, wobei der Prozessor bei Ausführung des Programmcodes dazu betreibbar ist, weitere Funktionen durchzuführen, darunter Funktionen für Folgendes:
Empfangen einer Eingabe, welche die Einstellung für den Verzögerungsparameter angibt; und
basierend auf der empfangenen Eingabe, Einstellen des Verzögerungsparameters auf einen numerischen Wert.

3. Nichtflüchtiges computerlesbares Medium nach Anspruch 1, wobei der Prozessor bei Ausführung des Programmcodes dazu betreibbar ist, weitere Funktionen durchzuführen, darunter Funktionen für Folgendes:
Empfangen einer Eingabe, welche die Einstellung für den Erweiterungsparameter angibt; und
basierend auf der empfangenen Eingabe, Einstellen des Erweiterungsparameters auf einen numerischen Wert.

4. Nichtflüchtiges computerlesbares Medium nach Anspruch 3, wobei der Prozessor bei Ausführung des Programmcodes dazu betreibbar ist, weitere Funktionen durchzuführen, darunter, wenn die Eingabe empfangen wird, welche die Einstellung für den Erweiterungsparameter angibt, Funktionen für Folgendes:
Empfangen von Blutzuckermesswerten über einen Zeitraum, der sich von vor bis nach dem Empfang der relativen Einnahmezeit der Mahlzeit erstreckt;
Bestimmen eines Verlaufs (398) der empfangenen Blutzuckermesswerte;
Vergleichen des bestimmten Verlaufs (398) mit bekannten Blutzuckermesswert-Verläufen bezüglich Mahlzeiten unterschiedlicher Zusammensetzung;
basierend auf einem Ergebnis des Vergleichs, Bestimmen, dass die Angabe zur allgemeinen Zusammensetzung der Mahlzeit genau war;
Bestätigen, dass die empfangene Eingabe, welche die Einstellung für den Erweiterungsparameter angibt, korrekt ist; und
basierend auf der Bestätigung, Nutzen der empfangenen Eingabe bei der Bestimmung des Erweiterungsparameters, wobei der Erweiterungsparameter eine weitere Zeitverzögerung zusätzlich zu dem Verzögerungsparameter ist.

5. Nichtflüchtiges computerlesbares Medium nach Anspruch 1, wobei der Prozessor bei Ausführung des Programmcodes dazu betreibbar ist, wenn die Einstellung für die Abgabebeschränkung bestimmt wird, weitere Funktionen durchzuführen, darunter Funktionen für Folgendes:
Empfangen einer Eingabe, welche die Einstellung für die Abgabebeschränkung angibt; und
basierend auf der empfangenen Eingabe, Einstellen der Abgabebeschränkung auf einen numerischen Wert.

6. Nichtflüchtiges computerlesbares Medium nach Anspruch 5, wobei der Prozessor bei Ausführung des Programmcodes dazu betreibbar ist, weitere Funktionen durchzuführen, darunter Funktionen für Folgendes:
Bestimmen, dass die empfangene Eingabe, welche die Einstellung für die Abgabebeschränkung angibt, eine Eingabe ist, die angibt, dass eine eingenommene Mahlzeit eine große Mahlzeit ist; und
Einstellen der Abgabebeschränkung auf einen sehr entspannten voreingestellten Prozentsatz über einer abzugebenden maximalen Insulinmenge.

7. Nichtflüchtiges computerlesbares Medium nach Anspruch 5, wobei der Prozessor bei Ausführung des Programmcodes dazu betreibbar ist, weitere Funktionen durchzuführen, darunter Funktionen für Folgendes:
Bestimmen, dass die empfangene Eingabe, welche die Einstellung für die Abgabebeschränkung angibt, eine Eingabe ist, die angibt, dass eine eingenommene Mahlzeit eine mittlere Mahlzeit ist; und
Einstellen der Abgabebeschränkung auf einen entspannten voreingestellten Prozentsatz über einer abzugebenden maximalen Insulinmenge.

8. Nichtflüchtiges computerlesbares Medium nach Anspruch 5, wobei der Prozessor bei Ausführung des Programmcodes dazu betreibbar ist, weitere Funktionen durchzuführen, darunter Funktionen für Folgendes:
Bestimmen, dass die empfangene Eingabe, welche die Einstellung für die Abgabebeschränkung angibt, eine Eingabe ist, die angibt, dass eine eingenommene Mahlzeit eine kleine Mahlzeit ist; und
Einstellen der Abgabebeschränkung auf einen typischen voreingestellten Prozentsatz über einer abzugebenden maximalen Insulinmenge.

9. Nichtflüchtiges computerlesbares Medium nach Anspruch 1, wobei der Prozessor, wenn der Programmcode ausgeführt wird, dazu betreibbar ist, weitere Funktionen durchzuführen, darunter, wenn das Mahlzeitmodell aus der Mehrzahl von Mahlzeitmodellen ausgewählt wird, Funktionen zum:
Bestimmen von Faktoren, die verwendbar sind, um eine Anzahl von Mahlzeitmodellen zur Auswahl aus der Mehrzahl von Mahlzeitmodellen zu begrenzen, wobei die Faktoren eine Tageszeit, eine Kalendereinstellung für einen Tag, an dem die Mahlzeit eingenommen wird, eine engste Übereinstimmung mit einem Mahlzeitprofilverlauf oder Benutzereingaben beinhalten;
Identifizieren von Mahlzeitmodellen aus der Mehrzahl von Mahlzeitmodellen, die eine größte Anzahl von Faktoren erfüllen;
Ausschließen aller Mahlzeitmodelle aus der Mehrzahl von Mahlzeitmodellen mit Ausnahme der identifizieren Mahlzeitmodelle; und
Wählen eines der identifizieren Mahlzeitmodelle zur Auswahl basierend auf einem Konfidenzniveau, das mit jedem der identifizierten Mahlzeitmodelle verknüpft ist.

10. Nichtflüchtiges computerlesbares Medium nach Anspruch 1, wobei der Prozessor bei Ausführung des Programmcodes dazu betreibbar ist, weitere Funktionen durchzuführen, darunter Funktionen für Folgendes:
Empfangen von Blutzuckermesswerten über einen Zeitraum, der sich von vor bis nach dem Empfang der relativen Einnahmezeit der Mahlzeit erstreckt;
Bestimmen eines Verlaufs (398) der empfangenen Blutzuckermesswerte;
Vergleichen des bestimmten Verlaufs (398) mit dem Verlauf eines Blutzuckermesswertes einer schnell aufnehmbaren Mahlzeit;
Quantifizieren eines Ähnlichkeitswertes einer schnellen Aufnahme zwischen dem bestimmten Verlauf und dem Verlauf eines Blutzuckermesswertes einer schnell aufnehmbaren Mahlzeit;
Vergleichen des bestimmten Verlaufs mit dem Verlauf eines Blutzuckermesswertes einer langsam aufnehmbaren Mahlzeit;
Quantifizieren eines Ähnlichkeitswertes einer langsamen Aufnahme zwischen dem bestimmten Verlauf und dem Verlauf eines Blutzuckermesswertes einer langsam aufnehmbaren Mahlzeit;
basierend auf einem höchsten Ähnlichkeitswert, Bestimmen des Erweiterungsparameters, wobei der Erweiterungsparameter eine weitere Zeitverzögerung zusätzlich zu dem Verzögerungsparameter ist.

11. Vorrichtung, umfassend:
einen Prozessor (461);
einen Speicher (463), der Programmcode - eine einer künstlichen Bauchspeicheldrüse zugehörigen Anwendung (469) - speichert, und der dazu betreibbar ist, Daten bezüglich der einer künstlichen Bauchspeicheldrüse zugehörigen Anwendung zu speichern, wobei der Programmcode und die einer künstlichen Bauchspeicheldrüse zugehörige Anwendung von dem Prozessor (461) ausgeführt werden können; und
wobei der Prozessor beim Ausführen der einer künstlichen Bauchspeicheldrüse zugehörigen Anwendung dazu betreibbar ist, die Insulinabgabe zu steuern und Funktionen auszuführen, darunter Funktionen für Folgendes:
Empfangen (210) von Informationen bezüglich der Einnahme einer Mahlzeit, wobei die Informationen eine grobe Angabe einer Größe der Mahlzeit, eine relative Einnahmezeit der Mahlzeit und eine Angabe zur allgemeinen Zusammensetzung der Mahlzeit beinhaltet;
Identifizieren (225) eines Blutzuckermesswertes, der innerhalb eines vorbestimmten Zeitbereichs der relativen Einnahmezeit der Mahlzeit empfangen wurde;
Auswählen (235) eines Mahlzeitmodells aus einer Mehrzahl von Mahlzeitmodellen basierend auf dem identifizierten Blutzuckermesswert;
Bestimmen (245) einer Einstellung für einen Verzögerungsparameter basierend auf der empfangenen relativen Einnahmezeit der Mahlzeit;
Bestimmen (250) einer Einstellung für einen Erweiterungsparameter basierend auf der empfangenen Angabe zur allgemeinen Zusammensetzung der Mahlzeit;
Bestimmen (255) einer Einstellung für eine Abgabebeschränkung basierend auf der empfangenen groben Angabe der Größe der Mahlzeit;
Modifizieren (260) des ausgewählten Mahlzeitmodells unter Verwendung der bestimmten Einstellung für den Verzögerungsparameter, der bestimmten Einstellung für den Erweiterungsparameter und der bestimmten Einstellung für die Abgabebeschränkung;
Verwenden (265) des modifizierten, ausgewählten Mahlzeitmodells, Bestimmen einer abzugebenden Insulindosis in Reaktion auf die empfangenen Informationen; und
Ausgeben (270) einer Abgabeanweisung an eine Medikamentenabgabevorrichtung, wobei die ausgegebene Anweisung eine bestimmte abzugebende Insulindosis angibt.

12. Vorrichtung nach Anspruch 11, ferner umfassend:
einen Sendeempfänger, der dazu betreibbar ist, Signale von einem Blutzuckersensor (404) zu empfangen und Signale an einen Blutzuckersensor (404) zu übertragen,
wobei der Sendeempfänger hierzu betreibbar ist:
Empfangen einer Mehrzahl von Blutzuckermesswerten, wobei die jeweiligen Blutzuckermesswerte in jeweiligen vorbestimmten Zeitintervallen empfangen werden; und
Bereitstellen der gemessenen Blutzuckerwerte für den Prozessor (461) und die einer künstlichen Bauchspeicheldrüse zugehörige Anwendung (469).

13. Vorrichtung nach Anspruch 11, ferner umfassend:
einen Sendeempfänger, der dazu betreibbar ist, Signale von einer Medikamentenabgabevorrichtung (402) zu empfangen und Signale an eine Medikamentenabgabevorrichtung (402) zu übertragen,
wobei der Sendeempfänger hierzu betreibbar ist:
Weiterleiten der Anweisung an die Medikamentenabgabevorrichtung (402), wodurch die Abgabe der bestimmten Insulindosis angewiesen wird.

## Revendications

1. Support non transitoire lisible par ordinateur pourvu d'un code de programmation exécutable par un processeur, le processeur, lors de l'exécution du code de programmation, étant utilisable pour assurer des fonctions, y compris des fonctions pour :
recevoir (210) des informations relatives à l'ingestion d'un repas, les informations comprenant une indication grossière d'une taille du repas, une heure relative d'ingestion du repas et une indication de la composition générale du repas ;
identifier (225) une valeur de mesure de la glycémie reçue dans un intervalle de temps prédéterminé par rapport à l'heure relative d'ingestion du repas ;
sélectionner (235) un modèle de repas parmi une pluralité de modèles de repas ;
déterminer (245) un réglage pour un paramètre de délai sur la base de l'heure relative d'ingestion du repas reçue ;
déterminer (250) un réglage pour un paramètre d'extension sur la base de l'indication de la composition générale du repas reçue ;
déterminer (255) un réglage pour une contrainte d'administration sur la base de l'indication grossière reçue de la taille du repas ;
modifier (260) le modèle de repas sélectionné en utilisant le réglage déterminé pour le paramètre de délai, le réglage déterminé pour le paramètre d'extension et le réglage déterminé pour la contrainte d'administration ;
en utilisant (265) le modèle de repas sélectionné modifié, déterminer une dose d'insuline à administrer en réponse aux informations reçues ; et
émettre (270) une instruction d'administration à un dispositif d'administration de médicaments, l'instruction émise indiquant une dose d'insuline à administrer déterminée.

2. Support lisible par ordinateur non transitoire selon la revendication 1, le processeur, lors de l'exécution du code de programmation, étant utilisable pour assurer des fonctions, y compris des fonctions pour :
recevoir une entrée indiquant le réglage du paramètre de délai ; et
sur la base de l'entrée reçue, régler le paramètre de délai sur une valeur numérique.

3. Support lisible par ordinateur non transitoire selon la revendication 1, le processeur, lors de l'exécution du code de programmation, étant utilisable pour assurer des fonctions, y compris des fonctions pour :
recevoir une entrée indiquant le réglage du paramètre d'extension ; et
sur la base de l'entrée reçue, régler le paramètre d'extension sur une valeur numérique.

4. Support lisible par ordinateur non transitoire selon la revendication 3, le processeur, lors de l'exécution du code de programmation, étant utilisable pour assurer d'autres fonctions, y compris des fonctions, lors de la réception de l'entrée indiquant le réglage du paramètre d'extension, pour :
recevoir des valeurs de mesure de la glycémie sur une période allant d'avant à après la réception de l'heure relative d'ingestion du repas ;
déterminer une trajectoire (398) des valeurs de mesure de la glycémie reçues ;
comparer la trajectoire déterminée (398) à des trajectoires connues de valeurs de mesure de la glycémie liées à des repas de compositions différentes ;
sur la base d'un résultat de la comparaison, déterminer que l'indication de la composition générale du repas était exacte ;
confirmer que l'entrée reçue indiquant le réglage du paramètre d'extension est correcte ; et
sur la base de la confirmation, utiliser l'entrée reçue dans la détermination du paramètre d'extension, le paramètre d'extension étant un délai supplémentaire qui s'ajoute au paramètre de délai.

5. Support lisible par ordinateur non transitoire selon la revendication 1, le processeur, lors de l'exécution du code de programmation, étant utilisable, lors de la détermination du réglage de la contrainte d'administration, pour réaliser d'autres fonctions, y compris des fonctions pour :
recevoir une entrée indiquant le réglage de la contrainte d'administration ; et
sur la base de l'entrée reçue, régler la contrainte d'administration sur une valeur numérique.

6. Support lisible par ordinateur non transitoire selon la revendication 5, le processeur, lors de l'exécution du code de programmation, étant utilisable pour assurer des fonctions, y compris des fonctions pour :
déterminer que l'entrée reçue indiquant le réglage de la contrainte d'administration est une entrée indiquant qu'un repas ingéré est un repas copieux ; et
régler la contrainte d'administration sur un pourcentage prédéfini très détendu au-dessus d'une quantité maximale d'insuline à administrer.

7. Support lisible par ordinateur non transitoire selon la revendication 5, le processeur, lors de l'exécution du code de programmation, étant utilisable pour assurer des fonctions, y compris des fonctions pour :
déterminer que l'entrée reçue indiquant le réglage de la contrainte d'administration est une entrée indiquant qu'un repas ingéré est un repas moyen ; et
régler la contrainte d'administration sur un pourcentage prédéfini détendu au-dessus d'une quantité maximale d'insuline à administrer.

8. Support lisible par ordinateur non transitoire selon la revendication 5, le processeur, lors de l'exécution du code de programmation, étant utilisable pour assurer des fonctions, y compris des fonctions pour :
déterminer que l'entrée reçue indiquant le réglage de la contrainte d'administration est une entrée indiquant qu'un repas ingéré est un petit repas ; et
régler la contrainte d'administration sur un pourcentage typique prédéfini au-dessus d'une quantité maximale d'insuline à administrer.

9. Support lisible par ordinateur non transitoire selon la revendication 1, le processeur, lors de l'exécution du code de programmation, étant utilisable pour assurer d'autres fonctions, y compris des fonctions, lors de la sélection du modèle de repas parmi la pluralité de modèles de repas, pour :
déterminer des facteurs utilisables pour limiter un nombre de modèles de repas à sélectionner parmi la pluralité de modèles de repas, les facteurs comprenant l'heure de la journée, le calendrier d'une journée au cours de laquelle le repas est ingéré, la correspondance la plus proche avec une trajectoire de profil de repas, ou des entrées de l'utilisateur ;
identifier des modèles de repas parmi la pluralité de modèles de repas qui satisfont le plus grand nombre de facteurs ;
éliminer tous les modèles de repas parmi la pluralité de modèles de repas à l'exception des modèles de repas identifiés ; et
choisir l'un des modèles de repas identifiés à sélectionner sur la base d'un niveau de confiance associé à chacun des modèles de repas identifiés.

10. Support lisible par ordinateur non transitoire selon la revendication 1, le processeur, lors de l'exécution du code de programmation, étant utilisable pour assurer des fonctions, y compris des fonctions pour :
recevoir des valeurs de mesure de la glycémie sur une période allant d'avant à après la réception de l'heure relative d'ingestion du repas ;
déterminer une trajectoire (398) des valeurs de mesure de la glycémie reçues ;
comparer la trajectoire déterminée (398) à une trajectoire de valeur de mesure de la glycémie d'un repas à absorption rapide ;
quantifier une valeur de similarité à absorption rapide entre la trajectoire déterminée et la trajectoire de la valeur de mesure de la glycémie du repas à absorption rapide ;
comparer la trajectoire déterminée à une trajectoire de mesure de la glycémie au cours d'un repas à absorption lente ;
quantifier une valeur de similarité à absorption lente entre la trajectoire déterminée et la trajectoire de la valeur de mesure de la glycémie du repas à absorption lente ;
sur la base d'une valeur de similarité la plus élevée, déterminer le paramètre d'extension, le paramètre d'extension étant un délai supplémentaire qui s'ajoute au paramètre de délai.

11. Dispositif, comprenant :
un processeur (461) ;
une mémoire (463) stockant un code de programmation, une application de pancréas artificiel (469), et étant utilisable pour stocker des données relatives à l'application de pancréas artificiel, le code de programmation et l'application de pancréas artificiel étant exécutables par le processeur (461) ; et
le processeur, lors de l'exécution de l'application de pancréas artificiel, étant utilisable pour commander l'administration de l'insuline et réaliser des fonctions, y compris des fonctions pour :
recevoir (210) des informations relatives à l'ingestion d'un repas, les informations comprenant une indication grossière d'une taille du repas, une heure relative d'ingestion du repas et une indication de la composition générale du repas ;
identifier (225) une valeur de mesure de la glycémie reçue dans un intervalle de temps prédéterminé par rapport à l'heure relative d'ingestion du repas ;
sélectionner (235) un modèle de repas parmi une pluralité de modèles de repas sur la base de la valeur de mesure de la glycémie identifiée ;
déterminer (245) un réglage pour un paramètre de délai sur la base de l'heure relative d'ingestion du repas reçue ;
déterminer (250) un réglage pour un paramètre d'extension sur la base de l'indication de la composition générale du repas reçue ;
déterminer (255) un réglage pour une contrainte d'administration sur la base de l'indication grossière reçue de la taille du repas ;
modifier (260) le modèle de repas sélectionné en utilisant le réglage déterminé pour le paramètre de délai, le réglage déterminé pour le paramètre d'extension et le réglage déterminé pour la contrainte d'administration ;
en utilisant (265) le modèle de repas sélectionné modifié, déterminer une dose d'insuline à administrer en réponse aux informations reçues ; et
émettre (270) une instruction d'administration à un dispositif d'administration de médicaments, l'instruction émise indiquant une dose d'insuline à administrer déterminée.

12. Dispositif selon la revendication 11, comprenant en outre :
un émetteur-récepteur utilisable pour recevoir des signaux d'un capteur de glycémie (404) et transmettre des signaux à ce dernier,
l'émetteur-récepteur étant utilisable pour :
recevoir une pluralité de valeurs de mesure de la glycémie, les valeurs de mesure de la glycémie respectives étant reçues à des intervalles de temps prédéterminés respectifs ; et
fournir les valeurs de glycémie mesurées au processeur (461) et à l'application de pancréas artificiel (469).

13. Dispositif selon la revendication 11, comprenant en outre :
un émetteur-récepteur utilisable pour recevoir des signaux d'un dispositif d'administration de médicaments (402) et transmettre des signaux à ce dernier,
l'émetteur-récepteur étant utilisable pour :
transmettre l'instruction au dispositif d'administration de médicaments (402) ordonnant l'administration de la dose déterminée d'insuline.
